# EUROPEAN PATENT SPECIFICATION

(11) **EP 1 649 278 B1**
(45) Date of publication and mention of the grant of the patent: **31.10.2007**
(21) Application number: 04801965.7
(22) Date of filing: 08.07.2004
(51) Int. Cl.: G01N 33/18

(54) **REMOTE CONTAMINATION MONITORING SYSTEM FOR WATER SUPPLY NETWORK**
FERNGESTEUERTES KONTAMINATIONSÜBERWACHUNGSSYSTEM FÜR WASSERVERSORGUNGSNETZWERK
SYSTEME DE SURVEILLANCE A DISTANCE POUR RESEAU D'ALIMENTATION EN EAU

(30) Priority: 11.07.2003 US 486368 P
(43) Date of publication of application: 26.04.2006
(73) Proprietor: PDA Security Solutions, Inc., SC 29651 (US)
(72) Inventor: PAGE, Daniel, V., Campobello, SC 29322 (US)
(74) Representative: von Kreisler, Alek
(86) International application number: PCT/US2004/021955
(87) International publication number: WO 2005/022145

(56) References cited:
- EP-B- 0 336 794
- WO-A-01/94937
- GB-A- 2 312 278
- US-A- 5 563 345
- US-A- 5 646 863
- US-A- 5 865 991
- US-A1- 2002 130 069
- US-A1- 2004 006 513
- US-B1- 6 245 224
- US-B2- 6 560 543

## Description

### BACKGROUND OF THE INVENTION

The present invention relates to an integral water testing and response system particularly useful with distributed water networks.

There has been a long felt desire to monitor water quality. This desire is particularly prevalent in water distribution systems such as municipal and regional water supply networks. For many decades water quality monitoring was strictly developed to thwart naturally occurring situations such as chlorine dissipation, stagnation and introduction of unintended materials from storm runoff, animal fecal matter, etc. These issues are still relevant but have now been relegated to secondary considerations in the light of potential terrorist activities wherein purposeful harm is anticipated.

Purposeful contamination, by terrorist or the like, creates particularly difficult problems with regards to detection and elimination. While a successful contamination could cause many people to become ill, or worse, the mere realization of an attempt would create social and economic havoc. Therefore, while detecting and decontaminating are critical, achieving this without mass hysteria is absolutely critical to a successful thwarting of an attempted attack.

Various systems are described wherein water quality is monitored remotely. Moskoff for example, in U.S. Pat. No. 6,753,186, describes a monitoring system wherein each individual residence is offered a detector. The detector relays quality information to a central monitoring station for comparison with standards. If the water quality is outside of standard conditions an alert is activated. While this system may be useful for detecting certain conditions it is contrary to current efforts to thwart terrorism. For example, an alteration of the water conditions upstream from a community can cause widespread panic due to large numbers of simultaneous alerts. The social and economic impact of sorting out the source, and severity, of the problem would constitute a successful attack even if no injury occurred.

Remote monitoring systems are known wherein data is transmitted to a process control system for control or verification purposes. Such a system is provided, for example, by Morton in U.S. Pat. No. 5,646,863.

In many prior art systems a detection of contamination occurs at the point of use while the alteration of the water occurs at the treatment plant. This can generate false signals and over-control situations. For example, if a low chlorine reading is observed in a low flow region efforts to respond to that signal may cause additional chlorine to be injected into the water at the treatment plant. Areas with high flow, or short residence times, could then experience excess chlorine which is unacceptable. Therefore, even though the low chlorine reading may be corrected a high reading may be indicated in other parts of the system.

It is also known to have remote control of a localized testing and maintenance system as provided by Enoki et al. in U.S. Pat. No. 6,245,224. This mitigates the problems of over-control but would be required at each customer site.
US 6,245,224 describes a water quality management system for managing the water quality in a water supply network having water pipe lines running form the purification plant to consumers to maintain the water quality at the consumers at an appropriate level. The water quality monitors for measuring the quality of water in the pipe lines are installed in the small-pipe water distribution network branching from the water distribution main pipe network and running toward the consumers. The water distribution facility for improving the water quality is installed at the base point of the small-pipe water distribution network. The water quality in the small-pipe water distribution network is managed based on the signals from the water quality monitors.

The prior art systems provide various forms of detection but, in the event of a purposeful contamination, the entire system is potentially compromised. There would be no indication of where the contamination occurs except that it is somewhere between the supply, or processing plant, and detector. The entire network of pipes between the detector and supply would therefore contain water which may, or may not, be contaminated. This problem is exasperated by dissipation of the contaminate since the entire system would eventually be contaminated even upstream of the site of contamination unless action is taken.

There has been a long felt desire for a system which can detect deviations in water quality, occurring either naturally or purposefully, and which is capable of isolating the impure water with minimal social and economic disruption. Such a system is provided herein.

### SUMMARY OF THE INVENTION

It is an object of the present invention to provide a detection and alert system for a distributed water supply.

It is another object of the present invention to provide an integrated system allowing for discrete detection and control of a distributed water supply network.

It is yet another object of the present invention to provide a contamination and detection control system which is capable of isolating any contamination with minimal disruption.

A particular feature of the present invention is the ability to generate, and monitor, a profile of attributes allowing for a response which is appropriate to the risk. This particular feature is particularly advantageous when automated risk management protocols are utilized.

It is another object of the present invention to provide a contamination detection system with remote detection and the option for local or central control of the various components of the system.

A particular feature of the present invention is the ability to couple access keys with the control system such that access to critical nodes can be controlled and monitored.

These and other advantages, as will be realized, are provided in a water quality detection system according to claim 1.

Another embodiment is provided in a method for determining contamination levels in flowing liquid according to claim 16.

### BRIEF SUMMARY OF DRAWINGS

Fig. 1 is a schematic representation of an embodiment of the present invention.

Fig. 2 is a schematic representation of another embodiment of the present invention.

Fig. 3 is a schematic representation of another embodiment of the present invention.

Fig. 4 is a diagrammatic representation of a preferred embodiment of the present invention.

Fig. 5 is a flow chart illustrating a preferred embodiment of the present invention.

### DETAILED DESCRIPTION OF THE INVENTION

The present invention is directed to a protected water distribution network wherein certain water transmission lines can be individually, or collectively, monitored and isolated automatically to substantially decrease the social, economic and political impact of purposeful tampering or contamination. The network also allows for automatic determination and isolation of non-purposeful upsets in water quality.

The invention will be described with reference to the figures forming an integral part of the instant specification. Throughout the specification similar elements will be numbered accordingly.

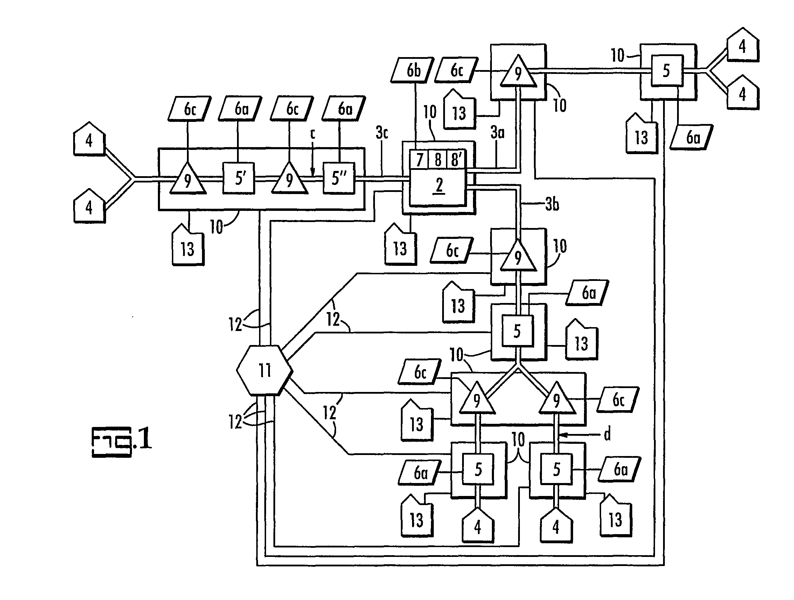

An embodiment of the network is illustrated in Fig. 1. The network, generally represented at 1, comprises a water distribution facility, 2, wherein water is processed to potable water for distribution through supply lines, 3, to consumers, 4. For the purposes of the present discussion three supply lines, 3a-c, are illustrated. It would be understood that the number of supply lines is not limited herein and that certain supply lines may form redundant supply lines to specific consumers.

The supply line, 3, has a detector, 5, integral thereto and capable of detecting at least one attribute of water flowing there through. Each detector comprises a communication link, 6a, which transmits the attribute from the detector, 5, to a central controller, 7, through a communication link, 6b. The central controller, 7, comprises a comparator, 8, capable of determining if the attribute is within limits considered to be acceptable. If the attribute is not considered to be within an acceptable range the central controller, 7, transmits a signal through the communication link, 6b, to at least one shut-off valve, 9, through a communication link, 6c, of the shut-off valve. The flow can be interrupted at the appropriate level without disruption of the entire network. The acceptable range of each attribute can be stored in the comparator, in a data storage unit, 8', as part of the comparator or at a remote site such as a government sponsored and supported database and retrievable through communication link 6b.

A particular feature of the present invention is the ability to control access of each node of the network. For the purposes of the present invention a node is a decision or action point such as a detector, a shut-off valve, a communication device or linkage, a distribution facility or the like. Each node comprises an access gate, 10, in communication with an access controller, 11, through a communication link, 12. An access key, 13, allows input of a personal attribute prior to entry into the access gate, 10. The personal attribute is specific to the individual and preferably comprises a biometric measurement such as a fingerprint, iris scan, optical or visual scan and voice recognition scan. The access controller, 11, determines if the personal attribute correlates to that of one with allowed access into the access gate and, if so, allows access. The access gate, and access there through, may be for a single node or multiple nodes as desired. Multiple nested access gates may be incorporated such as a key and lock access coupled with a biometric recognition. The access gates may be nested allowing for sequential access or multiple parameters for access.

A particular feature of the present invention is the ability to isolate discrete areas of the distribution network to minimize risk of contamination outside of those isolated areas. For example, referring specifically to supply line 3c, a series of detectors, 5, and shutoff valves, 9, allow discrete portions of the supply line to be isolated. For example, if a contaminant is entered at location "C" the first detection is likely to be at the next downstream detector, 5'. Each shut-off valve, 9, on either side of the detecting detector would then be automatically closed to isolate that portion between the closed shut-off valves. Alternatively, a detection would initiate a response protocol commensurate with the level and type of detection. If the upstream detector, 5", also detects a contamination the entire supply line, 3c, would be isolated at the water distribution facility, 2, to prevent diffusion back into the distribution plant. The contaminant would therefore be isolated and mitigated by flushing, chemical reaction, or other techniques as appropriate. While the consumer on supply line 3c may realize a disruption in service those consumers on lines 3 a and 3b would not be inconvenienced.

In yet another example, a contaminant entering the system at "D" would allow a secondary line to be isolated without disruption of other secondary lines branching off of the same main line. In this case the primary lines can be more adequately protected by physical protection and monitoring leaving only the secondary lines accessible to potential terrorist. If an attack occurs in a secondary line the impact is less severe and the number of potentially disrupted customers is limited. While automatic control of the shut-off valves is preferred, due to expediency, the automatic control may include an alert with human activation to automatic or physically activate the shut-off valve.

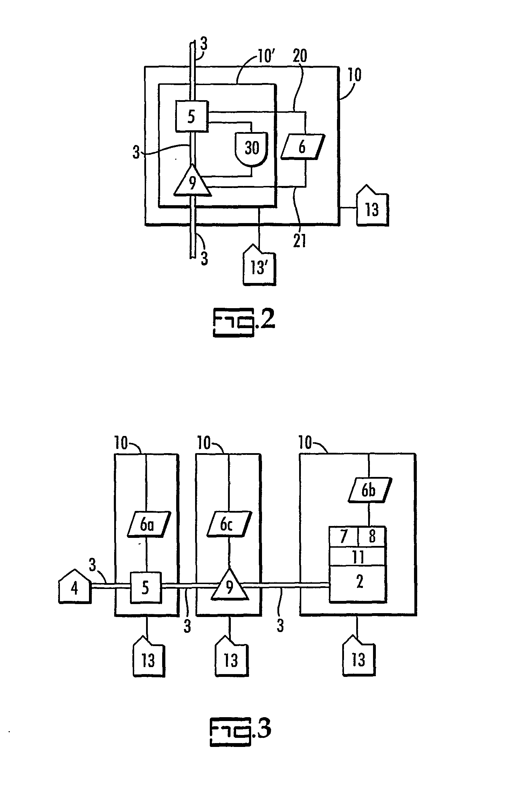

Another embodiment of the present invention is illustrated in Fig. 2. In Fig. 2, the supply line, 3, passes through a first node comprising a detector, 5, and a second node comprising a shut-off valve, 9. The detector, 5, transmits data through a communication linkage, 20, to a communication link, 6. As described previously, the communication link, 6, transmits data to a complimentary communication link integral to the central controller. The shut-off valve, 9, communicates with the communication link, 6, through a communication linkage, 21. In the embodiment illustrated in Fig. 2, the detector, 5, shut-off valve, 9, communication link, 6, and communication linkages, 20 and 21, are all within an access gate, 10. Nested within access gate, 10, is a second access gate, 10'. Access to the communication link, 6, only requires authentification of access key 13. Access to the detectors and shut-off valve requires authentification at access key 13 and access key 13'. As would be realized based on the disclosure herein access can be controlled in this manner such that certain individuals have access to portions of the protected area whereas other may have access to additional, or different, areas. A local controller, 30, is optionally provided to receive data from the detector, 5, and, if necessary, activate the shut-off valve, 9, based on predetermined criteria. The local controller may act as a secondary controller and only activate if communication is lost with a primary controller. Alternatively, the local controller can be a primary controller, either automatically or in response to human intervention, and any action taken thereby reported to a central controller through communication link, 6. In another embodiment the local controller can store data for later transmission to the central controller after a period of communication failure. The local controller may also communicate with the access key and act as a local access controller. It would be understood that water flow could be in either direction in Fig. 2 with either the detector or shut-off valve being upstream of the other.

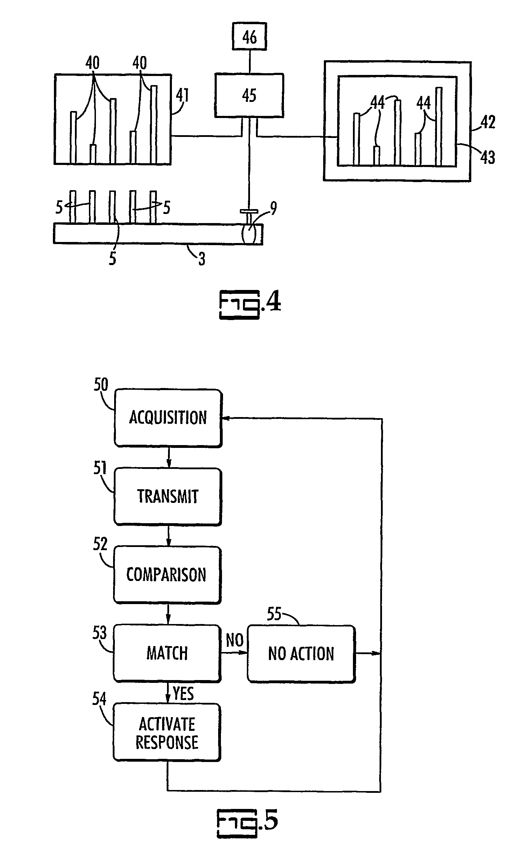

Yet another embodiment is illustrated in Fig. 3. In Fig. 3, the water distribution facility, 2, transmits water to a consumer, 4, through a supply line, 3. Associated with the supply line, 3, are a detector, 5, and shut-off valve, 9. The detector, 5, communicates via a communication link, 6a, with the central controller, 7, through a communication link, 6b. The central controller, 7, comprises a comparator, 8, and access controller, 11. The operation of the comparator and access controller are described further herein. An access key, 13, is associated with each access gate, 10. The access key communicates through the communication link of the particular node. In this preferred embodiment the node and communication link are within the access gate for added security.

A particular advantage of the present invention is the ability to cross-correlate data between detectors thereby allowing protection without the necessity for a full array of test at each detection node. Where appropriate complementary detectors can be used to correlate related data such as temperature and chlorine content, contamination with flow rate and temperature, organic content with pH or any combination that would be indicative of an upset in intended or unintended contents. Particularly useful correlated parameters are oxidation reduction and time which can be indicative of certain biological activity. This allows for the use of lower cost detectors and enhances the overall effectiveness at a lower cost. Single parameter or multiple parameter detectors can be utilized. Multiple parameter detectors are preferred due, in part, to simplified incorporation into a single access port. It is most preferred that multiple parameter detectors measure at least three attributes. It is more preferred that multiple parameter detectors measure at least five attributes. It is even more preferred that multiple parameter detectors measure at least nine attributes. A particular preferred detector measures free chlorine, oxidation-reduction potential, pH, temperature, specific conductance, dissolved oxygen and turbidity. A particularly preferred two parameter detector measures pH and temperature. For the purpose of the present disclosure "detector" refers to either a single parameter detector, a multiple parameter detector or one parameter detector of a multiple parameter detector.

The present system examines water quality parameters in order to detect any changes in the water quality profile of a given water supply. Examples of parameters measured include, but are not limited to: pH, temperature, time, conductivity, oxidation-reduction potential, salinity, turbidity, dissolved oxygen, alkalinity, hardness, color, manganese, copper, disinfectant residual, total phosphate, ortho phosphate, fluoride, free chlorine, tank level, chloride, corrosivity, total solids, total volatile solids, acidity, carbonate alkalinity, total suspended solids, sulfate, biological oxygen demand, chemical oxygen demand, bicarbonate alkalinity, total dissolved solids, sulfite, total coliform, fecal coliform, fecal streptococcus, heterotrophic bacteria, mold, giardia cysts, yeast, cryptosporidium oocysts, denitrifying bacteria, calcium, sodium, arsenic, cadmium, iron, mercury, silver, tin, magnesium, aluminum, barium, chromium, lead, nickel, strontium, vanadium, potassium, antimony, beryllium, copper, selenium, thallium, zinc, total organic carbon and other contaminants or parameters as desired. Particular preferred attributes include free chlorine, oxidation-reduction potential, pH, temperature, specific conductance, dissolved oxygen and turbidity. More preferred parameters include pH and temperature. Free chlorine is a particular preferred parameter. Parameter and attributes are used interchangeably herein to refer to measurements of water quality or water contaminants.

Examples of suitable detectors include: From ABB, 8002 Ammonia, 8232 Ammonia, AX410 Conductivity, AX411 Conductivity, AX413 Conductivity, AX416 Conductivity, AX460 pH/ORP, 8235 Chloride and 7976 Water Quality; from Campbell Scientific, Inc., OBS-3 Turbidity Monitor, 107 Thermister, 108 Thermister, CSIM11 pH Probe, CS511 Dissolved Oxygen Probe and CS547A Conductivity/Temperature Probe; from +GF+Signet, 515/2536 Paddlewheel Rotor-X Flow Sensor, 525 Metalex Flow Sensor, 2000 Micro Flow Sensor, 2100 Turbine Flow Sensor, 2507 Mini Flow Sensor, 2540/2541/2517 High Performance Flow Sensor, 2550/2560 Electromagnetic Flow Sensor, 7000/7001 Vortex Flow Sensor, 2764-2767 Differential DryLoc pH/ORP Electrodes, 2774-2777 Threaded DryLoc pH/ORP Electrodes, 2714/2715/2716/2717 pH & ORP Electrodes, 2754/2755/2756/2757 pH & ORP Electrodes, 2850 DryLoc^{™} Conductivity/Resistivity Sensor, 3719 pH/ORP Wet-Tap, 2819-2823 Conductivity/Resistivity Electrodes, 2839-2842 DryLoc^{™} Conductivity/Resistivity Electrodes, 2350 Temperature Sensor, 2450 Pressure Sensors and 2450 Pressure Sensors; from Electro-Chemical Devices Inc., DOS10, DOS17 and DOS17-VSS; from Metler-Toledo, Inc., HA405-DPA, HA405-DXK, HF405-DXK, InPro3030, InPro3100, InPro3200, InPro3200 SG, InPro4010, InPro4250, InPro4800, LoT406-M6-DXK, Pt4805-DPA, Pt4805, HA465-50-EQ-T-S7/9848, 465-50-SC-P-S7/9848, HA465-50-SC-T-S7, In Pro 2000, Pt-4865-50-SC-P-S7/9848, Pt-4865-50-SC-T-S7, InPro4501VP, InPro4550VP, InPro3300, InPro6050, InPro6800, InPro6900, InTap4000e, InTap4004e, InPro7010, InPro7000VP, InPro7001VP, InPro7002-TC-VP, InPro7005VP, InPro7108-25-VP, InPro7108-TC-VP, InPro7108-VP/CPVC, InPro7108-VP/PEEK, InPro7200, InPro7201, InPro7202, InPro8400, InPro8500, InPro8050, InPro8100, InPro8200/S(H)/Epoxy, InPro8200/S/Kalrez-FDA and InPro5000; from Endress+Hauser, Inc., Orbisint CPS11, CeraLiquid CPS41, CeraGel CPS71, OrbiPac GPS71, OrbiPore CPS91, PuriSys CPF201, TopHit CPS441, TopHit CPS471, TopHit CPS491, Orbisint CPS12, Cleanfit CPA451, Ecofit CPA640, CeraLiquid P CPS42, CeraGel P CPS72, ConduMax H CLS16, Condumax CLS21, ConduMax W CLS12, ConduMax W CLS13, ConduMax W CLS15, ConduMax W CLS 19, Indumax H CLS52, Indumax P CLS50, Smartec-S CLD132, Oxymax-W COS31, Oxymax-W COS41, Oxymax-W COS71, Oxymax H COS21, Trace Chlorine CCS141, Chlorine CCS140, Trace Chlorine dioxide CCS241, Chlorine dioxide CCS240, Turbimax CUS31, Turbimax CUS31E, Turbimax GUS31S, Turbimax-W CUS41 and TurbiMax CUS65; from Omega Engineering, Inc., ISE-8700, ISE-8800, ISE-8900, PHE-5580-20, PHE-5590-20, PHE-6300, PHE-5300, PHE-2114, PHE-1304, PHE-9153-15, PHE-9151-15_PHE-9152-15 and PHE-9150-15; from O.I. Analytical Model 1010 TOC Analyzer; from Rosemount Analytical, Model 381 ORP Rebuildable Sensor, Model 370 pH Sensor, Model 372 HF Resistant pH Sensor, Model 381 ph Rebuildable Sensor, Model 300 Retractable pH/ORP Sensor, Model 328A Steam Sterilizable pH Sensor, Model Hx338 Steam Sterilizable & Autoclavable pH Sensor, Model Hx348 Steam Sterilizable & Autoclavable pH Sensor, Model 396 Retraction/Submersion/Insertion pH/ORP Sensor, Model 396VP Retraction/Submersion/Insertion pH/ORP Sensor, Model TF396 Non-glass pH Sensor for Submersion/Insertion, Model 396P Retraction/Submersion/Insertion pH/ORP Sensor, Model 381+ Insertion/Submersion Flow Through Sensor, Model 381 PE Rebuildable Sensor, Model 385 pH/ORP Sensor - Retractable, Model 398 pH/ORP Sensor, Model 397 TUpH Sensor & the Quick-Loc Kit, Model 396R Retraction/Submersion/Insertion pH/ORP Sensor, Model 396RVP Retraction/Submersion/Insertion pH/ORP Sensor, Model 398R pH/ORP Sensor, Model 398RVP Retraction/Submersion/Insertion pH/ORP Sensor, Model 396PVP Submersion/Insertion pH/ORP Sensor, Model 398VP Retraction/Submersion/Insertion pH/ORP Sensor, Model 385+ Retractable/Submersion/Insertion pH/ORP Sensor, Model 389 pH/ORP Sensors, Model 389VP pH/ORP Sensors, Model 404 ENDURANCE^{™} Conductivity Sensors, Model 402 ENDURANCE^{™} Conductivity Sensors, Model 403 ENDURANCE^{™} Conductivity Sensors, Model 401 ENDURANCE^{™} Conductivity Sensors, Model 400 ENDURANCE^{™} Conductivity Sensors, Model 400VP ENDURANCE^{™} Conductivity Sensors, Model 141 Conductivity Sensor, Model 142 Conductivity Sensor, Model 150 Conductivity Sensor, Model 140 Conductivity Sensor, Model 402VP ENDURANCE^{™} Conductivity Sensors, Model 403VP ENDURANCE^{™} Conductivity Sensors, Model 225 Toroidal Conductivity Sensor, Model 247 Economy Toroidal Conductivity Sensor, Model 228 Insertion/Submersion Toroidal Conductivity Sensor and Model 226 Toroidal Conductivity Sensor; from In-Situ, Inc., TROLL 9000, MP TROLL 9000E, Flow-Sense Automated Low-Flow Sampling System and RDO; from Hach, 1153933-Conductivity Probe, 1648900-Conductivity Probe, 2930-Temperature Probe, 3700-Series, Analog Inductive Conductivity Sensors, PD1P1, PD1P3, PD2P1, PD3P1, PD1R1, PDIR3, DPD1P1, DPDIP3, DPD2P1, DPD3P1, DPD1R1, DPDIR3, DPS1, DRDIP5, DRD1P6, DRD2P5, DRD1R5, DRD1R6, DRS5, DPC1R1N, DPC1R1A, DPC1R2N, DPC1R2A , DPC1R3A, DPC2K1A, DPC2K2A, DPC3K2A, DRC1R5N, DRC2K5N, 1720E Low Range Process Turbidimeter Sensor(6010101), 3705E2T, 3706E2T, 3708E2T, 3725E2T, 3726E2T, 3727E2T, 3728E2T, 3422A1A, 3422A2A, 3422B3A, 3422C3A, 3422D3A, 3422E3A, 3433B8A, 3433E8A, 3444B8A, 3444D8A, 3455A6A, 3455C7A, 3455E7A, D3705E2T, D3706E2T, D3708E2T, D3725E2T, D3726E2T , D3727E2T, D3728E2T, D3422A1, D3422A2, D3422B3, D3422C3, D3422D3, D3422E3, D3433B8, D3433E8, D3444B8, D3444D8, D3455A6, D3455C7, D3455E7, Hach LDO Dissolved Oxygen Probe(5790000), 5740 sc Galvanic DO Sensor(5740DOB), 2200 PCX Online Particle Counting Sensor, HACH LDO^{™} Dissolved Oxygen Probe, PS4ABASE and HQ10-HQ20 Probes; from YSI, Inc., YSI 600LS Level Sonde, YSI 6600 EDS, YSI 6600 SONDE, YSI 6920 SONDE, YSI 600XLM SONDE, YSI 6820 SONDE, YSI 600XL SONDE, YSI 600R SONDE, 600 OMS - Optical Monitoring System, YSI 6025 Chlorophyll Sensor, YSI 6136 Turbidity Sensor and YSI 6130 Rhodamine WT Sensor; from Sensorex In-Line Mounted Electrodes, S660CD, S661CD, S662CD, S660CD, S661CD, S662CD, S660CD-ORP, S661CD-ORP, 970167, 970168, CS150, CS150TC, CS615, CS615TC-, CS620, CS620TC-, CS675, CS675TC-, CS676, CS676TC-, CS650, CS650TC-, CS675HP, CS675HPTC, CS676HP, CS676HPTC-, CS650HP, CS650HPTC, DO7000, DO7420 and DO6000; from Quantum Analytical Instruments, Q25P, Q22P, Q45P, Q25R, Q45R, Q25C4, Q45C4, Q25D and Q45D; from Analytical Sensors and Instruments Ltd., H061 and H068 VersaProbe; from APT Instruments, PH0300.515, PH0300.516, PH0300.468, PH0300.410, PH0300.411, WD35801.54, PH0300.400, PH0300.401, PH0300.420, PH0300.421, WD35801.54, PH0300.430, PH0300.431, PH0300.440, PH0300.441, WD35801.54, PH0300.450, PH0300.451, PH0300.452, PH0300.453, PH0300.750, PH0300.760 and PH0300.770; from AquaMetrix, Inc., P/R60C-8, P/R60-S, P/R60C-6, P/R60C-7 (Hot Tap), P/R65C, AM60/20 (Hot Tap), P91 and P91D; from Denver Instrument Company, Cat. No. 300728.1, Cat. No. 300729.1, Cat. No. 300738.1, Cat. No. 300731.1, Cat. No. 300736.1, Cat. No. 300737.1, Cat. No. 300735.1, Cat. No. 300733.1, Cat. No. 300739.1, Cat. No. 300742.1, Cat. No. 300762.1, Cat. No. 300743.1, Cat. No. 300744.1, Cat. No. 300745.1, Cat. No. 300741.1, Cat. No. 300746.1, Cat. No. 300740.1, Cat No. 301046.1, Cat. No. 301047.1, Cat. No. 301048.1 and Cat. No. 301058.1. ; from Hydrolab, Series 4a DataSonde Multiprobe, Series 4 DataSonde Miniprobe and Quanta Series; from Stevens Water Monitoring Systems, Inc., CTD 350, CTD 1200, CS 304 , CS4-1200, CTDP 300, CTDP 1200, DO 100, DO 300, DO 1200, EC 250, EC 350, EC 1200, pH 100, pH 300, pH 1200, TS 100, TS 300, TS1200, ORP 100, ORP 300 and ORP 1200; from Global Water Instrumentation, Inc, Temperature Sensor WQ101, pH Sensor WQ201, Conductivity Sensor WQ301, Dissolved Oxygen Sensor WQ401, ORP/Redux Sensor WQ600 and Turbidity Sensor WQ710; from Dascore Inc. Six-CENSE; from Instrumentation Northwest, Inc., PS9800 Pressure Transmitter, PS9801 Pressure Transducer, PS9805 Pressure Transducer, PS98i Pressure Transmitter, TempHion T-2, TempHion T-3, BV9000 and AquiStar^{®} PT2X; from Partech Instruments Ltd. Multi-Tech; and from Advanced Measurements and Control, Inc., CTD350, CTDP300, CS302 and CS304 and a ZAPS unit.

A particular feature of the present invention is the ability to utilize multiple detectors to create a profile of various contaminants. For example, certain potential contaminants may provide a unique signal under ultraviolet radiation while, at the same time, predictably altering pH, salinity and oxidation-reduction potential. Other contaminants, such as certain biological contaminants, may increase at higher temperature and low flow rates yet still be within acceptable levels. These may be naturally occurring phenomenon which can be mitigated by purging portions of a line and would otherwise not create a hazard. If the same contaminant increased at lower temperatures and lower flow rates this may indicate purposeful addition which requires an emergency response. Oxidation reduction potential as a function of time is a particularly useful indication of certain biological contaminants. Without correlated data the increase in the contaminant may be detected yet an adequate response may be lacking or inappropriate for the circumstances. For example a response appropriate for purposeful contamination may be employed to combat a natural phenomenon. In this instance the response itself could create chaos similar to that expected for an actual attack. By cataloging the profile of particular contaminants a library can be established for comparison with incoming data. If the profile of incoming data correlates sufficiently with a profile in the library the contaminant can be detected and proper precautions taken. A detection algorithm captures critical water parameter data and compares it to known chemical and biological signatures in a water profile database. By determining and profiling the type of organic substance, for example, the algorithm can classify the proper emergency response plan and notify the proper individuals defined by the system administrator along with water plant distribution administrators. One aspect of the emergency response plan may be an automatic valve shut-off procedure as set forth previously. The water profile database can be housed locally, networked, or available over a network such as the internet. The water profile database may be protected by passwords and/or biometrics. While contaminants are described herein it would be anticipated that the detection profile may include actual harmful materials as well as carriers, stabilizers, by products or other adjuvants related to harmful materials.

A diagrammatic representation of a preferred embodiment is provided in Fig. 4. In Fig. 4, a supply line, 3, has associated therewith a multiplicity of detectors, 5. Each detector generates at least one water attribute represented as a content signal, 40, in a data packet, 41. Each signal indicates the presence or level of measured attribute. A database, 42, comprises a multiplicity of stored data packets, 43, wherein each stored data packet represents known correlated representative signals, 44, consistent with a particular condition of the water. When the measured attributes have the same correlation as the correlated respresentative signal the particular condition of the water is known. A comparator, 45, compares the content signals, 40, of the data packet, 41, with the representative signals, 44, of the stored data packets, 43. If a match between the data packet reported by the detectors correlates with a stored data packet a response may be initiated such as transmission of a signal through a communication link, 46, whereby the shut-off valve, 9, may be activated automatically or by human intervention. It would be apparent that the data packets may comprise representative signals for a variety of conditions and that each representative signal may have a tolerance level within which the content signal must reside to consider the data to be a match. It is contemplated that the content signal may be compared individually or collectively. For example, a certain first content signal may be sufficient to cause an response regardless of the remaining content signals if above a certain level. At a second level the first content signal may only cause a response if a second content signal is also within a certain range. Each attribute may be compared at an absolute level or the ratio of levels for correlated attributes may be used for comparison with the correlated representative signals.

A preferred process of the present invention is illustrated in Fig. 5. In Fig. 5, data is acquired from an array of detectors at an acquisition step, 50. The data is transmitted, 51, to a comparator for a comparison, 52, with representative data. If a match is determined at 53, a response is activated, 54, commensurate with the conditions indicated by the detector or combination of detectors. If no match is determined at 53 no action is taken, 55. The cycle frequency from acquisition to match is determined based on the components and test rate necessity but is not particularly limiting herein. Each detector may sample at a predetermined rate and the data maintained for use in comparing with a combination of signals until a new data point is acquired. Alternatively, groups of detectors may utilize a similar sample frequency. Detectors with multiple attribute detection capabilities may sample all attributes simultaneously or on independent sample frequencies.

The communication link can be any communication link including twisted wire pairs, optical or terrestrial communication including hardwired communications and wireless communications. The communication links between communication devices are typically, but not limited to, a combination of transmission devices such as modems, fiber optical fibers, coaxial cable, twisted copper pairs, terrestrial signals relayed by satellites and/or antennas, phone lines, radio frequency transmissions, 802.11b Ethernet, BlueTooth and the like. Most commonly the communication link is a wide area network (or "WAN"), such as the internet or world wide web, which uses either public or private switching systems to form the communication linkages between various communication devices. The communication linkage is typically maintained and managed by service providers who provide a communication node whereby clients can link to the computer network through the communication node of the service provider for a predetermined fee. It is preferred that the communication link be transferred through a network. The term "network" as used herein refers specifically to a computer network. Computer networks, broadly speaking are a set of communication devices, or nodes, and communication links which interconnect the communication devices using standard protocols such as Hypertext Transport Protocol (HTTP) and Transmission Control Protocol/Internet Protocol (TCP/IP) or extensible markup language (XML) to form a network. The communication devices are typically computers, terminals, workstations, or other similar devices capable of receiving and/or sending data with each communication device being capable of residing at vastly different physical locations.

The term communication link refers herein to the transmission from one site to another whereas communication linkage refers to local communication. The terms may be used interchangeably in certain instances.

It is preferred that any communication link be encrypted as well known in the art. While not limited thereto a 128-minimum data encryption technique is preferred as are known encryption techniques such as SSL, IPSEC, Triple DES and the like.

Access control is typically monitored by at least one access key. Access keys include physical token based keys such as key and lock, smartcard technology, key pads by video surveillance by biometric analysis from a biometric scan or combinations thereof. Biometrics include, but are not limited to, fingerprint scan, iris scan, vascular scan and video scan. Examples of smartcard technology include, but are not limited to: stripe cards and proximity cards. Key pad administered access control technology includes password or passcode based systems. It is most preferred that at least one biometric analysis be included in the access control system. For the purposes of the present invention access key refers to the analysis of an authentication and access gate refers to all information which can only be accessed by passing the authentication including physical and digital locations and modalities. Video surveillance equipment includes, but is not limited to Pan-Tilt-Zoom (PTZ), Fixed, and Day/Night cameras, Infrared Illuminators, and Digital Video Multiplex Recorders (DVMRe).

A particularly preferred biometric analysis is fingerprint acquisition and comparison. There are many commercially available methods and techniques for fingerprint acquisition and comparison. While not limited thereto a particular preferred method is described in U.S. Pat. Appl. Publ. No. 2002/0054696 utilizing patterned floating electrodes. The fingerprint recognition device includes a transparent electrode layer to which one terminal of an AC power source is connected; a light emitting layer for forming an electric field between the transparent electrode layer and a finger forming a ground contact when being contacted with the finger and emitting light by this electric field for generating an optical fingerprint image according to ridge lines of a fingerprint image; a plurality of patterned floating electrodes arranged on the light emitting layer at a predetermined interval and turned on/off to output the optical fingerprint image; and a transparent insulating layer for transmitting the optical image generated from the light emitting layer.

The present invention has been described with particular reference to the preferred embodiments. It would be apparent to one of skill in the art that other embodiments, alterations and improvements could be envisioned based on the teachings herein without departing from the scope of the invention which is set forth in the claims appended hereto.

## Claims

1. A water quality detection system comprising:
a multiplicity of supply lines (3);
a multiplicity of detectors (5) wherein at least one detector of said detectors (5) is in each supply line of said supply lines (3) and is capable of monitoring at least one attribute of water and providing a signal related to said attribute;
a controller (7) capable of receiving each signal and comparing said signal to a control signal for said attribute;
a response mechanism (9),
**characterised by**
an access gate (10) limiting access to at least one of said detector (5), said controller(7) or said response mechanism (9);
an access key (13) for comparing a user attribute with a stored attribute wherein when said user attribute matches said stored attribute access is provided into said access gate (10),
and in that a response mechanism (9) is provided in each supply line (3) responsive to said controller (7) and activated when at least one signal from at least one detector (5) matches said control signal wherein said response mechanism (9) isolates water in said supply line (3) containing said detector (5) with said signal matching said control signal without disrupting flow in a second supply line (3).

2. The water quality detection system of claim 1 wherein said user attribute is a biometric.

3. The water quality detection system of claim 2 wherein said biometric is selected from fingerprint scan, iris scan, vascular scan and video scan.

4. The water quality detection system of claim 1 wherein said attribute is selected from pH, temperature, conductivity, oxidation-reduction, potential, salinity, turbidity, dissolved oxygen, alkalinity, hardness, color, manganese, copper, disinfectant residual, total phosphate, ortho phosphate, fluoride, chlorine, tank level, chloride, corrosivity, total solids, total volatile solids, carbonate alkalinity, total suspended solids, sulfate, biological oxygen demand, chemical oxygen demand, bicarbonate alkalinity, total dissolved solids, sulfite, total coliform, fecal coliform, fecal streptococcus, heterotrophic bacteria, mold, giardia cysts, yeast, cryptosporidium oocysts, denitrifying bacteria, calcium, sodium, arsenic, cadmium, iron, mercury, silver, tin, magnesium, aluminum, barium, chromium, lead, nickel, strontium, vanadium, potassium, antimony, beryllium, copper, selenium, thallium, zinc and total organic carbon.

5. The water quality detection system of claim 4 wherein said attribute is selected from pH, temperature, conductivity, oxidation-reduction potential, salinity, turbidity, dissolved oxygen, alkalinity, hardness, color, chlorine, tank level, chloride, total solids, biological oxygen demand, total coliform, fecal coliform, fecal streptococcus, heterotrophic bacteria, mold, giardia cysts, yeast, cryptosporidium oocysts, denitrifying bacteria, calcium, sodium, arsenic, cadmium, and total organic carbon.

6. The water quality detection system of claim 4 wherein said attribute is selected from free chlorine, oxidation-reduction potential, pH, temperature, specific conductance, dissolved oxygen and turbidity.

7. The water quality detection system of claim 6 wherein said attribute is selected from free chlorine, pH and temperature.

8. The water quality detection system of claim 7 wherein said attribute is selected from pH and temperature.

9. The water quality detection system of claim 1 wherein said response mechanism (9) is activated when multiple signals match multiple control signals.

10. The water quality detection system of claim 1 comprising multiple correlated control signals.

11. The water quality detection system of claim 1 wherein at least one detector (5) monitors multiple attributes.

12. The water quality detection system of claim 11 wherein at least one detector (5) monitors five or more attributes.

13. The water quality detection system of claim 1 wherein said response mechanism (9) comprises at least one shut-off valve.

14. The water quality detection system of claim 13 wherein said response mechanism (9) comprises at least two shut-off valves with said detector (5) between said shut-off valves.

15. The water quality detection system of claim 13 wherein said response mechanism (9) comprises automatic activation of at least one shut-off valve.

16. A method for determining contamination levels in flowing liquid by a water quality detection system according to claim 1 comprising:
measuring multiple distinct attributes of said flowing liquid by a detector (5) in a supply line (3);
comparing said multiple distinct attributes to correlated attributes wherein said correlated attributes indicate known contaminants and
activating a response when at least one comparative attribute of said multiple distinct attributes matches one comparative attribute of said correlated attributes,
**characterised by** that
the response is to isolate a contaminated portion of said flowing liquid in said supply line (3) containing said detector (5).

17. The method for determining contamination levels in flowing liquid of claim 16 wherein said attribute is selected from pH, temperature, conductivity, oxidation reduction potential, salinity, turbidity, dissolved oxygen, alkalinity, hardness, color, manganese, copper, disinfectant residual, total phosphate, ortho phosphate, fluoride, chlorine, tank level, chloride, corrosivity, total solids, total volatile solids, carbonate alkalinity, total suspended solids, sulfate, biological oxygen demand, chemical oxygen demand, bicarbonate alkalinity, total dissolved solids, sulfite, total coliform, fecal coliform, fecal streptococcus, heterotrophic bacteria, mold, giardia cysts, yeast, cryptosporidium oocysts, denitrifying bacteria, calcium, sodium, arsenic, cadmium, iron, mercury, silver, tin, magnesium, aluminum, barium, chromium, lead, nickel, strontium, vanadium, potassium, antimony, beryllium, copper, selenium, thallium, zinc and total organic carbon.

18. The method for determining contamination levels in flowing liquid of claim 17 wherein said attribute is selected from pH, temperature, conductivity, oxidation-reduction potential, salinity, turbidity, dissolved oxygen, alkalinity, hardness, color, chlorine, tank level, chloride, total solids, biological oxygen demand, total coliform, fecal coliform, fecal streptococcus, heterotrophic bacteria, mold, giardia cysts, yeast, cryptosporidium oocysts, denitrifying bacteria, calcium, sodium, arsenic, cadmium, and total organic carbon.

19. The method for determining contamination levels in flowing liquid of claim 18 wherein said attribute is selected from free chlorine, oxidation-reduction potential, pH, temperature, specific conductance, dissolved oxygen and turbidity.

20. The method for determining contamination levels in flowing liquid of claim 19 wherein said attribute is selected from free chlorine, pH and temperature.

21. The method for determining contamination levels in flowing liquid of claim 20 wherein said attribute is selected from pH and temperature.

22. The method for determining contamination levels in flowing liquid of claim 16 wherein multiple distinct attributes are measured by at least one detector (5).

23. The method for determining contamination levels in flowing liquid of claim 22 wherein at least one said detector (5) measures multiple distinct attributes.

24. The method for determining contamination levels in flowing liquid of claim 16 wherein said response comprises activating at least one shut-off valve.

25. The method for determining contamination levels in flowing liquid of claim 24 wherein said response comprises activating at least two shut-off valves with at least one detector (5) between said shut-off valves.

26. The method for determining contamination levels in flowing liquid of claim 24 wherein said response comprises automatic activation of at least one shut-off valve.

## Patentansprüche

1. Wasserqualitätsdetektiersystem mit:
mehreren Entnahmeleitungen (3);
mehreren Detektoren (5), wobei mindestens ein Detektor der Detektoren (5) in jeder Entnahmeleitung der Entnahmeleitungen (3) angeordnet ist und in der Lage ist, mindestens ein Merkmal des Wassers zu überwachen und ein auf dieses Merkmal bezogenes Signal zu erzeugen;
einem Controller (7) zum Empfangen jedes Signals und zum Vergleichen des Signals mit einem Kontrollsignal für das Merkmal;
einem Ansprechmechanismus (9),
**gekennzeichnet durch**
ein Zugriffsgatter (10) zum Begrenzen des Zugriffs auf den Detektor (5), den Controller (7) und/oder den Ansprechmechanismus (9);
einen Zugriffsschlüssel (13) zum Vergleichen eines Benutzermerkmals mit einem gespeicherten Merkmal, wobei bei Übereinstimmung des Benutzermerkmals mit dem gespeicherte Merkmal Zugriff auf das Zugriffsgatter (10) ermöglicht wird,
und **dadurch**, dass
ein Ansprechmechanismus (9) in jeder Entnahmeleitung (3) vorgesehen ist, der auf den Controller (7) anspricht und aktiviert wird, wenn mindestens ein Signal von mindestens einem Detektor (5) mit dem Kontrollsignal übereinstimmt, wobei der Ansprechmechanismus (9) mittels des mit dem Kontrollsignal übereinstimmenden Signals Wasser absperrt, das sich in der den Detektor (5) aufweisenden Entnahmeleitung (3) befindet, ohne den Strom in einer zweiten Entnahmeleitung (3) zu unterbrechen.

2. Wasserqualitätsdetektiersystem nach Anspruch 1, bei dem das Benutzermerkmal ein biometrisches Merkmal ist.

3. Wasserqualitätsdetektiersystem nach Anspruch 2, bei dem das biometrische Merkmal aus Fingerabdruckabtastung, Irisabtastung, Gefäßabtastung und Videoabtastung ausgewählt ist.

4. Wasserqualitätsdetektiersystem nach Anspruch 1, bei dem das Merkmal ausgewählt ist aus pH-Wert, Temperatur, Leitfähigkeit, Oxidationsreduktionspotential, Salzgehalt, Trübung, gelöstem Sauerstoff, Alkalinität, Härte, Farbe, Mangan, Kupfer, Desinfektionsmittelrückstand, Gesamtgehalt an Phosphat, Orthophosphat, Fluorid, Chlor, Behälterfüllstand, Chlorid, Korrosivität, Gesamtgehalt an Feststoffen, Gesamtgehalt an flüchtigen Feststoffen, Carbonatalkalinität, Gesamtgehalt an suspendierten Feststoffen, Sulfat, biologischem Sauerstoffbedarf, chemischem Sauerstoffbedarf, Bicarbonatalkalinität, Gesamtgehalt an gelösten Feststoffen, Sulfit, Gesamtgehalt an Coliform, Fäkalcoliform, Fäkalstreptokokken, heterotrophen Bakterien, Schimmel, Giardiazysten, Hefe, Cryptosporidium-Oozysten, denitrifizierenden Bakterien, Calcium, Natrium, Arsen, Cadmium, Eisen, Quecksilber, Silber, Zinn, Magnesium, Aluminium, Barium, Chrom, Blei, Nickel, Strontium, Vanadium, Kalium, Antimon, Beryllium, Kupfer, Selen, Thallium, Zink und Gesamtgehalt an organischem Kohlenstoff.

5. Wasserqualitätsdetektiersystem nach Anspruch 4, bei dem das Attribut ausgewählt ist aus pH-Wert, Temperatur, Leitfähigkeit, Oxidationsreduktionspotential, Salzgehalt, Trübung, gelöstem Sauerstoff, Alkalinität, Härte, Farbe, Chlor, Behälterfüllstand, Chlorid, Gesamtgehalt an Feststoffen, biologischem Sauerstoffbedarf, Gesamtgehalt an Coliform, Fäkalcoliform, Fäkalstreptokokken, heterotrophen Bakterien, Schimmel, Giardiazysten, Hefe, Cryptosporidium-Oozysten, denitrifizierenden Bakterien, Calcium, Natrium, Arsen, Cadmium und Gesamtgehalt an organischem Kohlenstoff.

6. Wasserqualitätsdetektiersystem nach Anspruch 4, bei dem das Attribut ausgewählt ist aus freiem Chlor, Oxidationsreduktionspotential, pH-Wert, Temperatur, spezifischem elektrischen Leitwert, gelöstem Sauerstoff und Trübung.

7. Wasserqualitätsdetektiersystem nach Anspruch 6, bei dem das Attribut aus freiem Chlor, pH-Wert und Temperatur ausgewählt ist.

8. Wasserqualitätsdetektiersystem nach Anspruch 7, bei dem das Attribut aus pH-Wert und Temperatur ausgewählt ist.

9. Wasserqualitätsdetektiersystem nach Anspruch 1, bei dem der Ansprechmechanismus (9) aktiviert wird, wenn mehrere Signale mit mehreren Kontrollsignalen übereinstimmen.

10. Wasserqualitätsdetektiersystem nach Anspruch 1 mit mehreren korrelierten Kontrollsignalen.

11. Wasserqualitätsdetektiersystem nach Anspruch 1, bei dem mindestens ein Detektor (5) mehrere Attribute überwacht.

12. Wasserqualitätsdetektiersystem nach Anspruch 11, bei dem mindestens ein Detektor (5) fünf oder mehr Attribute überwacht.

13. Wasserqualitätsdetektiersystem nach Anspruch 1, bei dem der Ansprechmechanismus (9) mindestens ein Absperrventil aufweist.

14. Wasserqualitätsdetektiersystem nach Anspruch 13, bei dem der Ansprechmechanismus (9) mindestens zwei Absperrventile aufweist, wobei der Detektor (5) zwischen den Absperrventilen angeordnet ist.

15. Wasserqualitätsdetektiersystem nach Anspruch 13, bei dem der Ansprechmechanismus (9) eine automatische Aktivierung mindestens eines Absperrventils umfasst.

16. Verfahren zum Bestimmen von Kontaminationspegeln in einer fließenden Flüssigkeit mittels eines Wasserqualitätsdetektiersystems nach Anspruch 1, mit folgenden Schritten:
Messen mehrerer unterschiedlicher Attribute der fließenden Flüssigkeit mittels eines in einer Entnahmeleitung (3) angeordneten Detektors (5);
Vergleichen der mehreren unterschiedlichen Attribute mit korrelierten Attributen, die bekannte Kontaminationsstoffe anzeigen; und
Aktivieren eines Ansprechens, wenn mindestens ein Vergleichsattribut der mehreren unterschiedlichen Attribute mit einem Vergleichsattribut der korrelierten Attribute übereinstimmt,
**dadurch gekennzeichnet, dass**
das Ansprechen dem Absperren eines kontaminierten Teils der fließenden Flüssigkeit in der den Detektor (5) aufweisenden Entnahmeleitung (3) dient.

17. Verfahren zum Bestimmen von Kontaminationspegeln in einer fließenden Flüssigkeit nach Anspruch 16, bei dem das Merkmal ausgewählt ist aus pH-Wert, Temperatur, Leitfähigkeit, Oxidationsreduktionspotential, Salzgehalt, Trübung, gelöstem Sauerstoff, Alkalinität, Härte, Farbe, Mangan, Kupfer, Desinfektionsmittelrückstand, Gesamtgehalt an Phosphat, Orthophosphat, Fluorid, Chlor, Behälterfüllstand, Chlorid, Korrosivität, Gesamtgehalt an Feststoffen, Gesamtgehalt an flüchtigen Feststoffen, Carbonatalkalinität, Gesamtgehalt an suspendierten Feststoffen, Sulfat, biologischem Sauerstoffbedarf, chemischem Sauerstoffbedarf, Bicarbonatalkalinität, Gesamtgehalt an gelösten Feststoffen, Sulfit, Gesamtgehalt an Coliform, Fäkalcoliform, Fäkalstreptokokken, heterotrophen Bakterien, Schimmel, Giardiazysten, Hefe, Cryptosporidium-Oozysten, denitrifizierenden Bakterien, Calcium, Natrium, Arsen, Cadmium, Eisen, Quecksilber, Silber, Zinn, Magnesium, Aluminium, Barium, Chrom, Blei, Nickel, Strontium, Vanadium, Kalium, Antimon, Beryllium, Kupfer, Selen, Thallium, Zink und Gesamtgehalt an organischem Kohlenstoff.

18. Verfahren zum Bestimmen von Kontaminationspegeln in einer fließenden Flüssigkeit nach Anspruch 17, bei dem das Attribut ausgewählt ist aus pH-Wert, Temperatur, Leitfähigkeit, Oxidationsreduktionspotential, Salzgehalt, Trübung, gelöstem Sauerstoff, Alkalinität, Härte, Farbe, Chlor, Behälterfüllstand, Chlorid, Gesamtgehalt an Feststoffen, biologischem Sauerstoffbedarf, Gesamtgehalt an Coliform, Fäkalcoliform, Fäkalstreptokokken, heterotrophen Bakterien, Schimmel, Giardiazysten, Hefe, Cryptosporidium-Oozysten, denitrifizierenden Bakterien, Calcium, Natrium, Arsen, Cadmium und Gesamtgehalt an organischem Kohlenstoff.

19. Verfahren zum Bestimmen von Kontaminationspegeln in einer fließenden Flüssigkeit nach Anspruch 18, bei dem das Attribut ausgewählt ist aus freiem Chlor, Oxidationsreduktionspotential, pH-Wert, Temperatur, spezifischem elektrischen Leitwert, gelöstem Sauerstoff und Trübung.

20. Verfahren zum Bestimmen von Kontaminationspegeln in einer fließenden Flüssigkeit nach Anspruch 19, bei dem das Attribut aus freiem Chlor, pH-Wert und Temperatur ausgewählt ist.

21. Verfahren zum Bestimmen von Kontaminationspegeln in einer fließenden Flüssigkeit nach Anspruch 20, bei dem das Attribut aus pH-Wert und Temperatur ausgewählt ist.

22. Verfahren zum Bestimmen von Kontaminationspegeln in einer fließenden Flüssigkeit nach Anspruch 16, bei dem mehrere unterschiedliche Attribute von mindestens einem Detektor (5) gemessen werden.

23. Verfahren zum Bestimmen von Kontaminationspegeln in einer fließenden Flüssigkeit nach Anspruch 22, bei dem mindestens ein Detektor (5) mehrere unterschiedliche Attribute misst.

24. Verfahren zum Bestimmen von Kontaminationspegeln in einer fließenden Flüssigkeit nach Anspruch 16, bei dem das Ansprechen das Aktivieren mindestens eines Absperrventils umfasst.

25. Verfahren zum Bestimmen von Kontaminationspegeln in einer fließenden Flüssigkeit nach Anspruch 24, bei dem das Ansprechen das Aktivieren von mindestens zwei Absperrventilen umfasst, wobei mindestens ein Detektor (5) zwischen den Absperrventilen angeordnet ist.

26. Verfahren zum Bestimmen von Kontaminationspegeln in einer fließenden Flüssigkeit nach Anspruch 24, bei dem das Ansprechen ein automatisches Aktivieren mindestens eines Absperrventils umfasst.

## Revendications

1. Système de détection de la qualité de l'eau, comprenant :
une multiplicité de conduits d'alimentation (3) ;
une multiplicité de détecteurs (5) dans laquelle au moins l'un desdits détecteurs (5) se trouve dans chacun desdits conduits d'alimentation (3) et est capable de contrôler au moins un attribut de l'eau et de fournir un signal en rapport avec ledit attribut ;
un dispositif de contrôle (7) capable de recevoir chaque signal et de comparer ledit signal à un signal témoin pour ledit attribut ;
un mécanisme de réponse (9),
**caractérisé par** :
un portail d'accès (10) limitant l'accès à au moins l'un dudit détecteurs (5), dudit dispositif de contrôle (7) ou dudit mécanisme de réponse (9) ;
une clé d'accès (13) pour comparer un attribut d'utilisateur avec un attribut stocké, où ledit attribut d'utilisateur qui concorde avec ledit accès d'attribut stocké est fourni dans ledit portail d'accès (10),
et ce qu'un mécanisme de réponse (9) est fourni dans chaque conduit d'alimentation (3) répondant audit dispositif de contrôle (7) et activé lorsqu'au moins un signal provenant d'au moins un détecteur (5) concorde avec ledit signal témoin, où ledit mécanisme de réponse (9) isole l'eau dudit conduit d'alimentation (3) contenant ledit détecteur (5) avec ledit signal concordant avec ledit signal témoin, sans perturber l'écoulement d'un deuxième conduit d'alimentation (3).

2. Système de détection de la qualité de l'eau selon la revendication 1, dans lequel ledit attribut d'utilisateur est un attribut biométrique.

3. Système de détection de la qualité de l'eau selon la revendication 2, dans lequel ledit attribut biométrique est choisi entre le balayage d'une empreinte digitale, le balayage de l'iris, le balayage vasculaire et le balayage vidéo.

4. Système de détection de la qualité de l'eau selon la revendication 1, dans lequel ledit attribut est choisi entre le pH, la température, la conductivité, le potentiel d'oxydoréduction, la salinité, la turbidité, l'oxygène dissous, l'alcalinité, la dureté, la couleur, le manganèse, le cuivre, les résidus de désinfectants, le phosphate total, l'orthophosphate, un fluorure, le chlore, le niveau du réservoir, un chlorure, la corrosivité, les solides totaux, les solides volatils totaux, l'alcalinité des carbonates, les solides totaux en suspension, un sulfate, la demande d'oxygène biologique, la demande d'oxygène chimique, l'alcalinité des bicarbonates, les solides dissous totaux, un sulfite, les agents coliformes totaux, les agents coliformes fécaux, les streptocoques fécaux, les bactéries hétérotrophes, les moisissures, les kystes de Giardia, une levure, des oocystes de Cryptosporidium, des bactéries dénitrifiantes, le calcium, le sodium, l'arsenic, le cadmium, le fer, le mercure, l'argent, l'étain, le magnésium, l'aluminium, le baryum, le chrome, le plomb, le nickel, le strontium, le vanadium, le potassium, l'antimoine, le béryllium, le cuivre, le sélénium, le thallium, le zinc et le carbone organique total.

5. Système de détection de la qualité de l'eau selon la revendication 4, dans lequel ledit attribut est choisi entre le pH, la température, la conductivité, le potentiel d'oxydoréduction, la salinité, la turbidité, l'oxygène dissous, l'alcalinité, la dureté, la couleur, le chlore, le niveau du réservoir, un chlorure, les solides totaux, la demande d'oxygène biologique, les agents coliformes totaux, les agents coliformes fécaux, les streptocoques fécaux, les bactéries hétérotrophes, les moisissures, les kystes de Giardia, une levure, des oocystes de Cryptosporidium, des bactéries dénitrifiantes, le calcium, le sodium, l'arsenic, le cadmium et le carbone organique total.

6. Système de détection de la qualité de l'eau selon la revendication 4, dans lequel l'attribut est choisi entre le chlore libre, le potentiel d'oxydoréduction, le pH, la température, la conductance spécifique, l'oxygène dissous et la turbidité.

7. Système de détection de la qualité de l'eau selon la revendication 6, dans lequel ledit attribut est choisi entre le chlore libre, le pH et la température.

8. Système de détection de la qualité de l'eau selon la revendication 7, dans lequel ledit attribut est choisi entre le pH et la température.

9. Système de détection de la qualité de l'eau selon la revendication 1, dans lequel ledit mécanisme de réponse (9) est activé lorsque de multiples signaux concordent avec de multiples signaux témoins.

10. Système de détection de la qualité de l'eau selon la revendication 1, comprenant de multiples signaux témoins en corrélation.

11. Système de détection de la qualité de l'eau selon la revendication 1, dans lequel au moins un détecteur (5) contrôle des attributs multiples.

12. Système de détection de la qualité de l'eau selon la revendication 11, dans lequel au moins un détecteur (5) contrôle cinq attributs ou davantage.

13. Système de détection de la qualité de l'eau selon la revendication 1, dans lequel ledit mécanisme de réponse (9) comprend au moins une soupape de coupure.

14. Système de détection de la qualité de l'eau selon la revendication 13, dans lequel ledit mécanisme de réponse (9) comprend au moins deux soupapes de coupure, ledit détecteur (5) étant situé entre lesdites soupapes de coupure.

15. Système de détection de la qualité de l'eau selon la revendication 13, dans lequel ledit mécanisme de réponse (9) comprend une activation automatique d'au moins une soupape de coupure.

16. Procédé pour déterminer les niveaux de contamination d'un liquide en écoulement par un système de détection de la qualité de l'eau selon la revendication 1, comprenant :
la mesure de multiples attributs distincts dudit liquide en écoulement par un détecteur (5) situé dans un conduit d'alimentation (3) ;
la comparaison desdits multiples attributs distincts avec des attributs en corrélation, lesdits attributs en corrélation indiquant les contaminants connus, et
l'activation d'une réponse lorsqu'au moins un attribut comparatif desdits multiples attributs distincts concorde avec un attribut comparatif desdits attributs en corrélation,
**caractérisé en ce que** :
la réponse consiste à isoler une partie contaminée dudit liquide en écoulement dans ledit conduit d'alimentation (3) contenant ledit détecteur (5).

17. Procédé de détermination des niveaux de contamination d'un liquide en écoulement selon la revendication 16, dans lequel ledit attribut est choisi entre le pH, la température, la conductivité, le potentiel d'oxydoréduction, la salinité, la turbidité, l'oxygène dissous, l'alcalinité, la dureté, la couleur, le manganèse, le cuivre, les résidus de désinfectants, le phosphate total, l'orthophosphate, un fluorure, le chlore, le niveau du réservoir, un chlorure, la corrosivité, les solides totaux, les solides volatils totaux, l'alcalinité des carbonates, les solides totaux en suspension, un sulfate, la demande d'oxygène biologique, la demande d'oxygène chimique, l'alcalinité des bicarbonates, les solides dissous totaux, un sulfite, les agents coliformes totaux, les agents coliformes fécaux, les streptocoques fécaux, les bactéries hétérotrophes, les moisissures, les kystes de Giardia, une levure, des oocystes de Cryptosporidium, des bactéries dénitrifiantes, le calcium, le sodium, l'arsenic, le cadmium, le fer, le mercure, l'argent, l'étain, le magnésium, l'aluminium, le baryum, le chrome, le plomb, le nickel, le strontium, le vanadium, le potassium, l'antimoine, le béryllium, le cuivre, le sélénium, le thallium, le zinc et le carbone organique total.

18. Procédé de détermination des niveaux de contamination d'un liquide en écoulement selon la revendication 17, dans lequel ledit attribut est choisi entre le pH, la température, la conductivité, le potentiel d'oxydoréduction, la salinité, la turbidité, l'oxygène dissous, l'alcalinité, la dureté, la couleur, le chlore, le niveau du réservoir, un chlorure, les solides totaux, la demande d'oxygène biologique, les agents coliformes totaux, les agents coliformes fécaux, les streptocoques fécaux, les bactéries hétérotrophes, les moisissures, les kystes de Giardia, une levure, des oocystes de Cryptosporidium, des bactéries dénitrifiantes, le calcium, le sodium, l'arsenic, le cadmium et le carbone organique total.

19. Procédé de détermination des niveaux de contamination d'un liquide en écoulement selon la revendication 18, dans lequel ledit attribut est choisi entre le chlore libre, le potentiel d'oxydoréduction, le pH, la température, la conductance spécifique, l'oxygène dissous et la turbidité.

20. Procédé de détermination des niveaux de contamination d'un liquide en écoulement selon la revendication 19, dans lequel ledit attribut est choisi entre le chlore libre, le pH et la température.

21. Procédé de détermination des niveaux de contamination d'un liquide en écoulement selon la revendication 20, dans lequel ledit attribut est choisi entre le pH et la température.

22. Procédé de détermination des niveaux de contamination d'un liquide en écoulement selon la revendication 16, dans lequel les multiples attributs distincts sont mesurés par au moins un détecteur (5).

23. Procédé de détermination des niveaux de contamination d'un liquide en écoulement selon la revendication 22, dans lequel au moins l'un desdits détecteurs (5) mesure les multiples attributs distincts.

24. Procédé de détermination des niveaux de contamination d'un liquide en écoulement selon la revendication 16, dans lequel ladite réponse comprend l'activation d'au moins une soupape de coupure.

25. Procédé de détermination des niveaux de contamination d'un liquide en écoulement selon la revendication 24, dans lequel ladite réponse comprend l'activation d'au moins deux soupapes de coupure, au moins un détecteur (5) étant situé entre lesdites soupapes de coupure.

26. Procédé de détermination des niveaux de contamination d'un liquide en écoulement selon la revendication 24, dans lequel ladite réponse comprend l'activation automatique d'au moins une soupape de coupure.
